# FASCICULE DE BREVET EUROPEEN

(11) **EP 0 725 057 B1**
(45) Date de publication et mention de la délivrance du brevet: **15.01.1997**
(21) Numéro de dépôt: 95119955.3
(22) Date de dépôt: 18.12.1995
(51) Int. Cl.: C07C 275/16, C07C 311/06, C07C 275/20, A61K 7/021, A61K 7/031, A61K 7/035, A61K 7/16, A61K 7/027, A61K 7/032, A61K 7/043, A61K 7/48

(54) **Dérivés de lysine à groupement alkylsulfonyle ou alkylaminocarbonyle, procédé de préparation, leur utilisation notamment en cosmétique et compositions les comprenant**
Alkylsulfonyl- oder alkylaminocarbonylsubstituierte Lysinderivate, Verfahren zu ihrer Herstellung, ihre Verwendung insbesondere in der Kosmetik und diese enthaltende Zusammensetzungen
Alkylsulfonyl or alkylaminocarbonyl substituted lysine derivatives, process for their preparation, their use especially in cosmetics and compositions containing them

(30) Priorité: 31.01.1995 FR 9501113
(43) Date de publication de la demande: 07.08.1996
(73) Titulaire: L'OREAL, 75008 Paris (FR)
(72) Inventeur: Bordier, Thierry, F-93290 Tremblay-en-France (FR); Philippe, Michel, F-91320 Wissous (FR)
(74) Mandataire: Dodin, Catherine

(56) Documents cités:
- EP-A- 0 139 481
- EP-A- 0 336 265
- EP-A- 0 447 287

## Description

La présente invention a pour objet des composés à groupement N^{ε}-alkylsulfonyle ou N^{ε}-alkylaminocarbonyle, dérivés de la lysine, leur procédé de préparation, leur utilisation notamment en cosmétique et les compositions, notamment cosmétiques, les comprenant.

On connaît des compositions, notamment cosmétiques, pharmaceutiques ou alimentaires, qui peuvent se présenter sous forme d'une poudre dite compacte, obtenue par compactage. Il s'agit généralement de compositions anhydres pouvant être constituées principalement de particules solides et d'un liant gras, mises en forme par compression.
L'élaboration de telles compositions soulève toutefois de nombreuses difficultés car la composition finale doit être suffisamment homogène et compacte pour présenter une bonne aptitude au prélèvement et pour éviter par ailleurs une fragmentation pouvant être provoquée notamment par des chocs.

Il est décrit, dans la demande de brevet EP139481, des compositions cosmétiques utilisant comme agents pour modifier la surface de composés inorganiques, en vue d'en augmenter la dispersibilité, soit un dérivé monoacylé d'un acide aminé basique dont le groupement acyle aliphatique a 8-22 atomes de carbone, soit un dérivé N,N-diacylé d'un acide aminé basique dont les groupements acyles identiques ou différents, ont 1-22 atomes de carbone.
Il est également décrit, dans la demande de brevet EP336265, des compositions cosmétiques pour la mise en forme des cheveux comprenant comme agents tensioactifs, un dérivé N-monoacylé d'un acide aminé basique dont le groupement acyle a 8-22 atomes de carbone.
On constate toutefois que les dérivés acylés des acides aminés basiques décrits précédemment sont très difficilement compactables, voire intassables.

L'invention a pour but de proposer de nouveaux composés, qui permettent de faciliter l'obtention de telles compositions, tout en satisfaisant aux exigences précitées, sans présenter les inconvénients de l'art antérieur.

La présente invention a donc pour objet un dérivé de la lysine à groupement N^{ε}-alkylsulfonyle ou N^{ε}-alkylaminocarbonyle de formule (I)

HOOC - CH (NH₂) - (CH₂)₄ - NH - X

dans laquelle X représente un groupement -SO₂R ou -CONHR, où R représente un radical alkyle linéaire ou ramifié, saturé ou insaturé, ayant de 8 à 22 atomes de carbone,
les sels des composés de formule (I) ainsi que leurs isomères optiques de configuration D ou L, et leurs mélanges.

Un autre objet de l'invention est une composition, entre autre cosmétique, pharmaceutique, hygiénique ou alimentaire, comprenant au moins un dérivé de formule (I).
Encore un objet de l'invention est l'utilisation d'au moins un dérivé de formule (I) comme substance de revêtement de particules substrat.
On a en effet constaté que lesdites particules, généralement des poudres, présentaient un toucher amélioré lorsqu'elles étaient revêtues dudit dérivé.

On a de plus constaté que les dérivés selon l'invention permettaient également de conférer à la composition cosmétique les comprenant des qualités d'étalement, d'adhésion à la peau et de diffusion de la lumière particulièrement intéressantes, ainsi qu'un toucher onctueux et agréable, et une résistance à l'eau améliorée.

Plus précisément, l'invention a pour objet un dérivé de la lysine de formule (I) :

HOOC - CH (NH₂) - (CH₂)₄ - NH - X

dans laquelle X représente un groupement - SO₂R ou - CONHR, où R représente un radical alkyle linéaire ou ramifié, saturé ou insaturé ayant de 8 à 22 atomes de carbone,
les sels des composés de formule (I) ainsi que leurs isomères optiques de configuration D ou L, et leurs mélanges.

Le radical R peut de préférence représenter un radical alkyle, linéaire ou ramifié ayant de préférence 8 à 16 atomes de carbone.
Parmi les dérivés selon l'invention, on peut citer la N^{ε}-dodécanesulfonyl-L-lysine et la N^{ε}-dodécylaminocarbonyl-L-lysine.

Les sels des composés de formule (I) peuvent être choisis parmi les sels de cations inorganiques monovalents, tels que ceux de sodium, ou divalents tels que ceux de zinc ou de cuivre. Les sels peuvent être aussi choisis parmi les sels de cations organiques tels que ceux d'aminopropanediol, de trishydroxyaminométhane, de glucamine et de N-méthylglucamine.

Les dérivés selon l'invention peuvent se présenter sous forme solide ayant une taille des particules comprise entre 10 - 500 000 nm, de préférence comprise entre 100 - 25000 nm.
Ces dérivés sont généralement peu solubles dans les huiles et dans les solutions aqueuses dont le pH est compris entre 5 et 8. Ils présentent généralement un point de fusion élevé supérieur à 200°C.

La composition selon l'invention comprenant lesdits dérivés peut se présenter sous diverses formes telles que des dispersions, des lotions éventuellement épaissies ou gélifiées, des poudres éventuellement « compactées », des laits, des crèmes, des stick, des mousses ou des sprays lorsqu'elle est conditionnée en aérosol, des émulsions huile-dans-eau ou eau-dans-huile, de dispersions liposomiales ou encore de préparations solides.
Les dérivés selon l'invention peuvent être compris dans la composition en une proportion allant de 0,05% à 80% en poids, de préférence en une proportion de 0,5 à 30% en poids par rapport au poids total de la composition.
Les dérivés selon l'invention peuvent être présents dans la composition sous forme libre et/ou sous forme d'une association avec des particules substrats qu'ils enrobent.

Outre le dérivé selon l'invention, la composition peut aussi comprendre au moins un additif choisi dans le groupe constitué par les agents tensioactifs, les corps gras, les solvants organiques, les silicones, les épaississants, les adoucissants, les filtres solaires, les agents de traitement, les agents anti-mousse, les agents hydratants, les parfums, les conservateurs, les agents antioxydants, les séquestrants, les agents de sapidité, les agents alcalinisants ou acidifiants, les charges et les pigments.
Parmi les corps gras utilisables dans la composition selon l'invention, on peut citer les huiles, les cires, les acides gras, les alcools gras et/ou leur mélange.
Les huiles et les cires peuvent être d'origine animale, végétale, minérale ou de synthèse. On peut citer, parmi les huiles, l'huile de palme hydrogénée, l'huile de ricin hydrogénée, l'huile de vaseline, l'huile de paraffine, l'huile de purcellin.
Parmi les cires, on peut citer la cire d'abeille, la cire de montan, la cire de Carnauba, la cire de candelilla, la cire de canne à sucre, la cire du Japon, l'ozokérite, les cires microcristallines, la cire de paraffine, la cire de lanoline, la cire de lanoline hydrogénée et la cire de lanoline acétylée.

Parmi les particules pouvant être enrobées par les dérivés selon l'invention, on peut citer notamment les pigments, les charges particulaires et les microsphères telles que les microsphères creuses de copolymères de chlorure de vinylidène/acrylonitrile. Plus particulièrement, on peut citer, parmi les charges, les charges insolubles éventuellement colorées telles que des nanopigments d'oxydes métalliques comme les oxydes de titane, de zinc, de fer, de manganèse, de césium et/ou de zirconium.

La composition selon l'invention peut se présenter sous forme de compositions de maquillage telles que des fonds de teint, des crèmes teintées, des mascaras, des fards à joues et à paupières, des rouges à lèvres, des vernis à ongles.
Selon une forme particulière de réalisation, elle peut se présenter sous forme dite compacte, le dérivé selon l'invention facilitant le compactage des ingrédients desdites compositions ainsi que la cohésion du produit compacté ce qui évite un effritement facile dudit produit compacté. Parmi ces compositions compactes, on peut citer notamment les fonds de teint, les fards à joues ou à paupières et les rouges à lèvres.

La composition selon l'invention peut également se présenter sous forme d'une composition pharmaceutique ou hygiénique telles que des pâtes dentifrices, des compositions exfoliantes, des poudres pour le corps ou pour bébés et des poudres antitranspirantes, voire sous forme d'une composition alimentaire.

L'invention a également pour objet un procédé de préparation d'un dérivé de formule (I), consistant :
- à faire réagir en milieu aqueux et à pH basique, de la lysine ou l'un de ses sels, de configuration connue, avec une solution d'un sel de cuivre,
- à faire réagir sur la solution du complexe de cuivre un composé de formule (II) choisi parmi

   R - SO₂ - Cl (IIa)

   R - NH - CO - Y (IIb)

   R - N = C = O (IIc)

   dans laquelle R représente un radical alkyle linéaire ou ramifié, saturé ou insaturé ayant de 8 à 22 atomes de carbone, et Y est choisi parmi les dérivés d'azole, comme le radical imidazolyle, ledit composé de formule (II) étant additionné avec ou sans solvant,
- à traiter le sel de cuivre de la lysine substituée obtenu par un agent décomplexant et, éventuellement
- à purifier le composé obtenu.
Parmi les solutions de sels de cuivre utilisées dans le procédé selon l'invention, on peut citer en particulier les solutions de sulfate de cuivre.
Le pH basique du milieu réactionnel, est de préférence compris entre 8 et 14.
Selon un mode de réalisation préférée du procédé de préparation, l'agent décomplexant utilisé est une solution aqueuse du sel disodique de l'acide éthylènediamine-tétracétique ou d'un acide tel que l'acide chlorhydrique.

On va maintenant donner à titre illustratif des exemples de préparation de dérivés de la lysine à groupement N^{ε}-alkylsulfonyle ou N^{ε}-alkylaminocarbonyle selon l'invention ainsi qu'un exemple de composition comprenant un tel dérivé.

### EXEMPLE 1 : Préparation de la N^{ε}-dodécanesulfonyl-L-lysine

Dans un tricol de 250 ml muni d'un thermomètre et d'une ampoule à introduction de 50 ml, 10 g de L-lysine monochlorhydrate sont dissous dans 44 ml d'une solution aqueuse de soude à 10%.

Une solution de 6,8 g de sulfate de cuivre pentahydraté dans 40 ml d'eau est introduite dans le milieu réactionnel.
Au mélange homogène refroidi à la température de 5°C, 4,6 g d'hydrogénocarbonate de sodium sont ajoutés suivi par l'addition goutte à goutte de chlorure de dodécanesulfonyle en solution dans le THF.
Après une nuit sous agitation à température ambiante, le milieu réactionnel est filtré et le précipité correspondant au produit final sous forme de complexe de cuivre, est lavé à l'eau et à l'acétone, puis séché à l'étuve sous pression réduite.
Pour éliminer le cuivre, le complexe est traité au reflux pendant 4 heures par une solution aqueuse de sel disodique dihydraté de l'acide éthylènediaminetétraacétique à 10%. Le traitement peut être reconduit plusieurs fois pour obtenir un produit totalement décomplexé.
On obtient 14 g (rendement 68%) de produit blanc nacré.

L'analyse chimique donne les caractéristiques suivantes :
- Fusion : décomposition à partir de 260°C (mesurée au bankofler)
- Spectre de masse : m/z 379,2 (MH+); 361,1 ; 335,2 ; 316,0
- Analyse élémentaire :(C18H38N2O4S, poids moléculaire 378,579)

| | C | H | N | O | S |
|---|---|---|---|---|---|
| % calculé | 57,11 | 10,12 | 7,4 | 16,9 | 8,47 |
| % mesuré | 56,84 | 10,05 | 7,28 | 16,98 | 8,72 |

- Granulométrie (mesurée par diffraction de la lumière au moyen d'un Leeds & Northrup modèle Microtrac X 100) : à 0,4 % dans un mélange équimolaire eau/éthanol on obtient, en nombre (moyenne) : 4,72 µm
- Chromatographie sur couche mince : HPCCM (silice Merck 60F254), avec comme éluant, un mélange NH₄OH 6 / CH₃OH 47 / CH₂Cl₂ 47
On obtient un rapport frontal Rf = 0,44.

### EXEMPLE 2 : Préparation de la N^{ε}-dodécylaminocarbonyl-L-lysine

Dans un tricol de 250 ml muni d'un thermomètre et d'une ampoule à introduction de 50 ml, 10 g de L-lysine monochlorhydrate sont dissous dans 44 ml d'une solution aqueuse de soude à 10%. Une solution de 6,8 g de sulfate de cuivre pentahydraté dans 40 ml d'eau est introduite dans le milieu réactionnel.
A température ambiante, une solution de 15,3 g de N-dodécylaminocarbonyl imidazole dans 80 ml de THF est ajoutée.
Après une nuit sous agitation à température ambiante, le milieu réactionnel est filtré et le précipité correspondant au produit final sous forme de complexe de cuivre, est lavé à l'eau et à l'acétone, puis séché à l'étuve sous pression réduite.
Pour éliminer le cuivre, le complexe est traité au reflux pendant 4 heures par une solution aqueuse de sel disodique dihydraté de l'acide éthylènediaminetétraacétique à 10%. Le traitement peut être reconduit plusieurs fois pour obtenir un produit totalement décomplexé.
On obtient 31 g (rendement 79%) d'un produit légèrement bleuté.

L'analyse chimique donne les caractéristiques suivantes :
- Fusion : T > 260°C (bankofler)
- Spectre de masse : m/z 358 (MH+) ; 186
- Analyse élémentaire (C19H39N3O3; poids moléculaire 357,541)

| | C | H | N | O |
|---|---|---|---|---|
| % calculé | 63,83 | 10,99 | 11,75 | 13,42 |
| % mesuré | 63,67 | 11,02 | 11,37 | 13,32 |

- Granulométrie (mesurée par diffraction de la lumière) : à 0,4 % dans un mélange équimolaire eau/éthanol on obtient, en nombre (moyenne) 0,59µm
- Chromatographie sur couche mince : HPCCM (silice Merck 60F254), éluant : mélange NH₄OH 6 / CH₃OH 47 / CH₂Cl₂ 47
On obtient : Rapport frontal Rf = 0,36

### EXEMPLE 3

On prépare une poudre compactée ayant la composition suivante :

| *Composition A :* | |
|---|---|
| - Talc | 38,4 g |
| - Oxychlorure de bismuth | 10 g |
| - Stéarate de zinc | 4 g |
| - Composé de l'exemple 1 | 20 g |
| - Poudre de Nylon | 20 g |

| *Composition B :* | |
|---|---|
| - Oxydes de fer | 1,6 g |
| - Huile de vaseline | 6 g |

La poudre est obtenue de la façon suivante : on broie la composition A dans un broyeur de type KENWOOD pendant environ 5 minutes sous faible agitation, on ajoute la composition B et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles.
On obtient une poudre compactée présentant une bonne adhésion, qui s'étale bien et de manière agréable sur la peau, tout en étant douce au toucher.
De plus, la présence du composé de l'exemple 1 évite un effritement trop facile du produit compacté.

## Revendications

1. Dérivé de la lysine à groupement N^{ε}-alkylsulfonyle ou N^{ε}-alkylaminocarbonyle de formule (I)
HOOC - CH (NH₂) - (CH₂)₄ - NH - X
dans laquelle X représente un groupement -SO₂R ou -CONHR, où R représente un radical alkyle linéaire ou ramifié, saturé ou insaturé, ayant de 8 à 22 atomes de carbone,
les sels des composés de formule (I) ainsi que leurs isomères optiques de configuration D ou L, et leurs mélanges.

2. Dérivé selon la revendication 1, caractérisé en ce que les sels sont choisis parmi les sels de cations inorganiques monovalents ou divalents et les sels de cations organiques.

3. Dérivé selon l'une des revendications précédentes, caractérisé en que le radical R représente un radical alkyle, linéaire ou ramifié, ayant de 8 à 16 atomes de carbone.

4. Dérivé selon l'une des revendications précédentes, caractérisé en ce qu'il est choisi parmi la N^{ε}-dodécanesulfonyl-L-lysine et la N^{ε}-dodécylaminocarbonyl-L-lysine.

5. Dérivé selon l'une des revendications précédentes, caractérisé en ce qu'il a une taille de particule comprise entre 10 et 500 000 nm.

6. Composition caractérisée en ce qu'elle comprend au moins un dérivé selon l'une des revendication 1 à 5.

7. Composition selon la revendication 6, caractérisée en ce que le dérivé de la lysine est compris entre 0,05 et 80% en poids par rapport au poids total de la composition.

8. Composition selon la revendication 7, caractérisée en ce que le dérivé de la lysine est compris en une proportion allant de 0,5 à 30% en poids par rapport au poids total de la composition.

9. Composition selon l'une des revendications 6 à 8, caractérisée en ce qu'elle comprend le dérivé de la lysine sous forme libre et/ou sous forme d'une association avec des particules substrat qu'il enrobe.

10. Composition selon la revendication 9, dans laquelle les particules substrats sont choisies parmi les pigments, les charges particulaires et les microsphères telles que les microsphères creuses de copolymères de chlorure de vinylidène/acrylonitrile.

11. Composition selon l'une des revendications 6 à 10, se présentant sous forme d'une composition cosmétique, pharmaceutique, hygiénique ou alimentaire.

12. Composition selon l'une des revendications 6 à 11, se présentant sous forme de fond de teint, mascara, fard à joues et à paupières, rouge à lèvres, vernis à ongles, composition exfoliante, pâte dentifrice, poudre pour le corps ou pour bébés et/ou poudre antitranspirante.

13. Procédé de préparation d'un dérivé selon l'une des revendications 1 à 5, caractérisé en ce qu'il consiste à faire réagir en milieu aqueux et à pH basique, de la lysine ou l'un de ses sels de configuration connue, avec une solution d'un sel de cuivre, puis à faire réagir sur la solution du complexe de cuivre un composé de formule (II) choisi parmi :
R - SO₂ - Cl (IIa)
R - NH - CO - Y et (IIb)
R - N = C = O (IIc)
dans laquelle R représente un radical alkyle linéaire ou ramifié, saturé ou insaturé ayant de 8 à 22 atomes de carbone, et Y est choisi parmi les dérivés d'azole, puis à traiter le sel de cuivre de lysine obtenu par un agent décomplexant.

14. Utilisation d'au moins un dérivé selon l'une des revendications 1 à 5 en tant que revêtement de particules substrat.

15. Utilisation d'au moins un dérivé selon l'une des revendications 1 à 5 pour faciliter le compactage de compositions comprenant des particules.

## Claims

1. Lysine derivative containing an N^{ε}-alkylsulphonyl or N^{ε}-alkylaminocarbonyl group of formula (I)
HOOC-CH(NH₂)-(CH₂)₄-NH-X
in which X represents an -SO₂R or -CONHR group, where R represents a linear or branched, saturated or unsaturated, alkyl radical having from 8 to 22 carbon atoms,
the salts of the compounds of formula (I), and their optical isomers of D or L configuration, and their mixtures.

2. Derivative according to Claim 1, characterized in that the salts are chosen from the salts of monovalent or divalent inorganic cations and the salts of organic cations.

3. Derivative according to one of the preceding claims, characterized in that the R radical represents a linear or branched alkyl radical having from 8 to 16 carbon atoms.

4. Derivative according to one of the preceding claims, characterized in that it is chosen from N^{ε}-dodecylsulphonyl-L-lysine and N^{ε}-dodecylamino-carbonyl-L-lysine.

5. Derivative according to one of the preceding claims, characterized in that it has a particle size of between 10 and 500,000 nm.

6. Composition, characterized in that it comprises at least one derivative according to one of Claims 1 to 5.

7. Composition according to Claim 6, characterized in that the lysine derivative is between 0.05 and 80 % by weight with respect to the total weight of the composition.

8. Composition according to Claim 7, characterized in that the lysine derivative is included in a proportion ranging from 0.5 to 30 % by weight with respect to the total weight of the composition.

9. Composition according to one of Claims 6 to 8, characterized in that it comprises the lysine derivative in the free form and/or in the form of a combination with substrate particles which it coats.

10. Composition according to Claim 9, in which the substrate particles are chosen from pigments, particulate fillers and microspheres such as hollow vinylidene chloride/acrylonitrile copolymer microspheres.

11. Composition according to one of Claims 6 to 10, which is provided in the form of a cosmetic, pharmaceutical, hygiene or food composition.

12. Composition according to one of Claims 6 to 11, which is provided in the form of a foundation, mascara, blusher, eye shadow, lipstick, nail varnish, exfoliative composition, toothpaste, powder for the body or for babies, and/or anti-perspirant powder.

13. Process for the preparation of a derivative according to one of Claims 1 to 5, characterized in that it consists in reacting, in aqueous medium and at basic pH, lysine or one of its salts, of known configuration, with a solution of a copper salt, in then reacting the solution of the copper complex with a compound of formula (II) chosen from:
R-SO₂-Cl (IIa)
R-NH-CO-Y and (IIb)
R-N=C=O (IIc)
in which R represents a linear or branched, saturated or unsaturated, alkyl radical having from 8 to 22 carbon atoms and Y is chosen from the azole derivatives, and in then treating the lysine copper salt obtained with a decomplexing agent.

14. Use of at least one derivative according to one of Claims 1 to 5, as coating for substrate particles.

15. Use of at least one derivative according to one of Claims 1 to 5, for facilitating compaction of compositions comprising particles.

## Patentansprüche

1. Lysinderivate mit N^{ε}-Alkylsulfonylgruppen oder N^{ε}-Alkylaminocarbonylgruppen der Formel (I)
HOOC-CH(NH₂)-(CH₂)₄-NH-X (I)
worin X eine SO₂R- oder eine CONHR-Gruppe bedeutet und R eine geradkettige oder verzweigte, gesättigte oder ungesättigte Alkylgruppe mit 8 bis 22 Kohlenstoffatomen ist,
die Salze der Verbindungen der Formel (I), ihre optischen Isomere in D- oder L-Konfiguration und deren Gemische.

2. Derivate nach Anspruch 1, dadurch gekennzeichnet, daß die Salze unter Salzen von einwertigen oder zweiwertigen anorganischen Kationen und Salzen von organischen Kationen ausgewählt sind.

3. Derivate nach einem der vorhergehenden Ansprüche, dadurch gekennzeichnet, daß die Gruppe R eine lineare oder verzweigte Alkylgruppe mit 8 bis 16 Kohlenstoffatomen bedeutet.

4. Derivate nach einem der vorhergehenden Ansprüche, dadurch gekennzeichnet, daß sie unter N^{ε}-Dodecansulfonyl-L-lysin und N^{ε}-Dodecylaminocarbonyl-L-lysin ausgewählt sind.

5. Derivate nach einer der vorhergehenden Ansprüche, dadurch gekennzeichnet, daß sie eine Partikelgröße im Bereich von 10 nm bis 500000 nm aufweisen.

6. Zusammensetzung, dadurch gekennzeichnet, daß sie mindestens ein Derivat nach einem der Ansprüche 1 bis 5 enthält.

7. Zusammensetzung nach Anspruch 6, dadurch gekennzeichnet, daß das Lysinderivat in Anteilen von 0,05 bis 80 Gew.-%, bezogen auf das Gesamtgewicht der Zusammensetzung, enthalten ist.

8. Zusammensetzung nach Anspruch 7, dadurch gekennzeichnet, daß das Lysinderivat in Anteilen von 0,5 bis 30 Gew.-%, bezogen auf das Gesamtgewicht der Zusammensetzung, enthalten ist.

9. Zusammensetzung nach einem der Ansprüche 6 bis 8, dadurch gekennzeichnet, daß sie das Lysinderivat in freier Form und/oder in Form einer Kombination mit Substratpartikeln, die es umhüllt, enthält.

10. Zusammensetzung nach Anspruch 9, in der die Substratpartikel ausgewählt sind unter Pigmenten, speziellen Füllstoffen und Mikrokügelchen, wie hohlen Mikrokügelchen von Vinylidenchlorid-Acrylnitril-Copolymeren.

11. Zusammensetzung nach einem der Ansprüche 6 bis 10, die in Form einer kosmetischen, pharmazeutischen Zusammensetzung oder Zusammensetzung zur Hygiene oder einer Lebensmittelzusammensetzung vorliegt.

12. Zusammensetzung nach einem der Ansprüche 6 bis 11, die als Make up, Mascara, Wangenrouge, Lidschatten, Lippenstift, Nagellack, Peelingzusammensetzung, Zahnpasta, Körperpuder oder Babypuder und/oder Antitranspirantpulver vorliegt.

13. Verfahren zur Herstellung eines Derivats nach einem der Ansprüche 1 bis 5, dadurch gekennzeichnet, daß es darin besteht, Lysin oder eines seiner Salze bekannter Konfiguration mit einer Kupfersalzlösung und anschließend die Lösung des Kupferkomlexes mit einer Verbindung der Formel (II) umzusetzen, die unter
R-SO₂-Cl, (IIA)
R-NH-CO-Y und (IIb)
R-N=C=O (IIc)
ausgewählt ist, worin R eine geradkettige oder verzweigte, gesättigte oder ungesättigte Alkylgruppe mit 8 bis 22 Kohlenstoffatomen bedeutet, und Y unter den Azolderivaten ausgewählt ist, und anschließend das Kupfersalz von Lysin mit einem Dekomplexierungsmittel zu behandeln.

14. Verwendung mindestens eines Derivats nach einem der Ansprüche 1 bis 5 als Überzug von Substratpartikeln.

15. Verwendung mindestens eines Derivats nach einem der Ansprüche 1 bis 5 zu Erleichterung der Verdichtung von Zusammensetzungen, die Partikel enthalten.
